# EUROPÄISCHE PATENTSCHRIFT

(11) **EP 1 434 559 B1**
(45) Veröffentlichungstag und Bekanntmachung des Hinweises auf die Patenterteilung: **03.12.2008**
(21) Anmeldenummer: 02782821.9
(22) Anmeldetag: 04.10.2002
(51) Int. Cl.: A61K 8/34, A61K 8/37, A61K 8/39, A61K 8/44, A61K 8/46, A61K 8/60, A61K 8/73, A61Q 5/02, A61Q 19/10, A61P 17/16

(54) **KOSMETISCHE UND/ODER PHARMAZEUTISCHE ZUBEREITUNGEN**
COSMETIC AND/OR PHARMACEUTICAL PREPARATIONS
PREPARATIONS COSMETIQUES ET/OU PHARMACEUTIQUES

(30) Priorität: 13.10.2001 DE 10150730
(43) Veröffentlichungstag der Anmeldung: 07.07.2004
(73) Patentinhaber: Cognis IP Management GmbH, 40589 Düsseldorf (DE)
(72) Erfinder: SEIPEL, Werner, 40723 Hilden (DE); NALBORCZYK, Mirella, 52445 Titz (DE); NIEENDICK, Claus, 47807 Krefeld (DE); BECKER, Anke, 40468 Dèusseldorf (DE)
(86) Internationale Anmeldenummer: PCT/EP2002/011106
(87) Internationale Veröffentlichungsnummer: WO 2003/032934

(56) Entgegenhaltungen:
- EP-A- 0 282 289
- US-A- 4 708 813
- US-A- 5 089 531

## Beschreibung

### Gebiet der Erfindung

Die Erfindung betrifft tensidische Zubereitungen mit einem Gehalt an Fruchtsäureestem und Fettalkoholen sowie die Verwendung von Fruchtsäureestem als Rückfettungsmittel in tensidischen Zubereitungen.

### Stand der Technik

Zubereitungen, die zur Reinigung und Pflege der menschlichen Haut und der Haare eingesetzt werden, enthalten in der Regel ein oder mehrere oberflächenaktive Substanzen, insbesondere auf Basis von anionischen oder amphoteren Tensiden. Da die alleinige Verwendung von Tensiden Haut und Haare zu sehr austrocknen würde, ist es im allgemeinen üblich, solchen Mitteln rückfettende Substanzen zuzusetzen. Tensidische Formulierungen sollen jedoch einerseits eine reinigende Wirkung haben, andererseits trotzdem einen rückfettenden Schutzfilm auf der Haut zurücklassen. Insbesondere bei "rinseoff Formulierungen besteht die Schwierigkeit, dass auch rückfettende Substanzen abgespült werden und in unzureichendem Maße ihre Wirkung entfalten können. Der Austausch von unerwünschten Fettbestandteilen auf der Haut, die dem Reinigungsprozeß unterliegen sollen, gegen rückfettende Bestandteile, die auf der Haut verbleiben sollen oder sich in die oberen Hautschichten einlagem sollen unterliegt einem empfindlichen Gleichgewicht.
In der US-Patentschrift US 5 089 531 werden Salze von Citronensäuremonoestem als hautglättende und feuchtigkeitshaltende Komponente offenbart. Der Rückfettungseffekt ist jedoch bei diesen Zubereitungen nicht zufriedenstellend. Gegenstand des US-Patentes US 6 024 947 sind "rinse-off"-Formulierungen mit einer Ölphase und Citronensäureestem, die hier dazu führen, dass die ölhaltige Formulierung besser abspülbar ist. Bei diesen Formulierungen werden zugunsten der Reinigungswirkung die Rückfettungseigenschaften vernachlässigt.
Die Deutsche Patentschrift DE 19621681 C2 hat wässrige Perlglanzkonzentrate zum Gegenstand, in denen hydroxysubstituierte Carbonsäureester als Perlglanzkomponente eingesetzt werden.

Die Aufgabe der Erfindung hat darin bestanden, tensidische Formulierungen mit gutem Reinigungseffekt bei gleichzeitiger gegenüber dem Stand der Technik verbesserter Rückfettungswirkung zur Verfügung zu stellen, die ein angenehmes Hautgefühl hinterlassen, eine gute dermatologische Verträglichkeit aufweisen und sich einfach herstellen lassen. Die in den Zubereitungen eingesetzten Rückfetter sollen dabei nicht viskositätsreduzierend sein.

### Beschreibung der Erfindung

Gegenstand der Erfindung sind kosmetische und/oder pharmazeutische Zubereitungen, enthaltend
(a) 0,1 bis 30 Gew.-% Fruchtsäureester ausgewählt aus Trialkylesterder Citronensäure und Dialkylester der Apfelsäure und
(b) 0,1 bis 90 Gew.-% anionische und/oder nichtionische und/oder kationische und/oder amphotere und/oder zwitterionische Tenside und
(c) 0,1 bis 10 Gew.-% Fettalkohole
   mit der Maßgabe, dass sich die Mengenangaben mit Wasser und gegebenenfalls üblichen Hilfs- und Zusatzstoffen zu 100 Gew.-% ergänzen, sowie die Verwendung von Fruchtsäureestem als Rückfetter in tensidischen Formulierungen.

Überraschenderweise wurde gefunden, dass die Kombination von Fruchtsäureestem mit Tensiden zu "rinse-off"-Formulierungen mit sehr guter Reinigungswirkung und gleichzeitigen optimalen rückfettenden Eigenschaften führt. Die Mischungen hinterlassen ein angenehmes Hautgefühl und weisen eine besonders hohe dermatologische Verträglichkeit auf. Schaumvolumen und Schaumstabilität sind trotz guter Rückfettungswirkung nicht vermindert.
Bei der Anwendung in Haarpftegemittein wird eine Verbesserung der Kämmbarkeit und Frisierbarkeit und ein angenehmer Weichgriff erzielt. Durch den Zusatz von Fettalkoholen kann die Verarbeitung der Mischungen verbessert werden, ohne dass trotz Veränderung der Lipophilie/Hydrophilie-Balance das Gleichgewicht zwischen Reinigungswirkung und Rückfettungswirkung verändert wird. Die Formulierungen lassen sich kalt verarbeiten und durch ihre flüssige Struktur leicht gleichmäßig verteilen.

### Fruchtsäureester

Bei den ausgewählten Fruchtsäureestem handelt es sich um bekannte Stoffe, die nach den einschlägigen Verfahren der präparativen organischen Chemie hergestellt werden können.
Die Säurekomponente wird mit gesättigten und ungesättigten langkettigen und verzweigten Alkoholen mit einer Kettenlänge von 8 bis 22 Kohlenstoffatomen, bevorzugt 16 bis 18 Kohlenstoffatomen und besonders bevorzugt 18 Kohlenstoffatomen verestert.
Trialkylester der Citronensäure und Dialkylester der Äpfelsäure werden eingesetzt. Speziell bevorzugt werden Citronensäuretrioctadecylester und Äpfelsäuredioctadecylester
Diese werden in Mengen von 0,1 bis 30 Gew.%, bevorzugt 0,5 bis 25 und besonders bevorzugt 1 bis 20 Gew. % in den erfindungsgemäßen Mitteln eingesetzt.

### Tenside

Als oberflächenaktive Stoffe können nichtionische, anionische, kationische und/oder amphotere bzw. zwitterionische Tenside enthalten sein, deren Anteil an den Mitteln üblicherweise bei etwa 0,1 bis 90 und vorzugsweise 5 bis 70 Gew.-% und besonders bevorzugt 10 bis 50 Gew. % beträgt. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)-sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acyl-aspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Alkyl- und/oder Alkenyloligoglykoside

Alkyl- und Alkenyloligoglykoside, die als besonders bevorzugte Tenside eingesetzt werden, stellen bekannte nichtionische Tenside dar, die der Formel (I) folgen,

**R¹O-[G]p** **(I**)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden, beispielsweise durch säurekatalysierte Acetalisierung von Glucose mit Fettalkoholen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyl-oligoglykosid eine analytisch ermittelte rechnerische Größe, die meistens eine gebrochene Zahl dar-stellt. Vorzugsweise werden Alkyl- und/oder Alkenyloligoglykoside mit einem mittleren Oligomeri-sierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palm-oleylalkohol, Stearylalkohol, Isostearylalkohol, Oleylalkohol, Elaidylalkohol, Petroselinylalkohol, Arachyl-alkohol, Gadoleylalkohol, Behenylalkohol, Erucylalkohol, Brassidylalkohol sowie deren technische Ge-mische, die wie oben beschrieben erhalten werden können. Bevorzugt sind Alkyloligoglucoside auf Basis von gehärtetem C_{12/14}-Kokosalkohol mit einem DP von 1 bis 3.

Alkyl- und/oder Alkenyloligoglycoside werden in den erfindungsgemäßen Mitteln zu 0,1 bis 90 Gew.%, bevorzugt 5 bis 70 Gew.% und besonders bevorzugt 10 bis 50 Gew.% eingesetzt.

### Fettalkohole

Unter Fettalkoholen sind primäre aliphatische Alkohole der Formel (II) zu verstehen,

**R²OH** **(II)**

in der R² für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, lsostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 10 bis 18 Kohlenstoffatomen, wie beispielsweise Kokos-, Palm-, Palmkern- oder Talgfettalkohol. Um das Gleichgewicht zwischen Reinigungseffekt und Rückfettungswirkung nicht zu stören, werden besonders bevorzugt Fettalkohole mit 12 bis 14 Kohlenstoffatomen eingesetzt.
Diese werden in Mengen von 0,1 bis 10 Gew.%, bevorzugt 0,2 bis 8 und besonders bevorzugt 0,5 bis 5 Gew. % in den erfindungsgemäßen Mitteln eingesetzt.

### Gewerbliche Anwendbarkeit

Die erfindungsgemäßen Zubereitungen zeichnen sich durch eine hohe Reinigungskapazität und ausgezeichnete rückfettende Eigenschaften aus. Sie sind gut dermatologisch verträglich, flüssig und pumpbar und lassen sich bei Zugabe von Fettalkoholen kalt verarbeiten. Sie weisen verdickende Eigenschaften auf und sind dabei vollständig biologisch abbaubar.
Ein weiterer Gegenstand der Erfindung betrifft daher ihre Verwendung von Fruchtsäureestem als Rückfettungsmittel in tensidischen Formulierungen, beispielsweise für den Bereich der Haar- und Körperpflege. Erfindungsgemäße Ausführungsformen der rückfettenden Zubereitungen enthalten:
a) 0,1 - 30 Gew. % Fruchtsäureester
b) 1 - 90 Gew. % anionische und/oder nichtionische und/oder kationische und/oder amphotere und/oder zwitterionische Tenside und
c) 0,1-10 Gew. % Fettalkohole,
insbesondere
a) 0,5 - 25 Gew. % Fruchtsäureester,
b) 5 - 70 Gew. % anionische und/oder nichtionische und/oder kationische und/oder amphotere und/oder zwitterionische Tenside und
c) 0,2 - 8 Gew. % Fettalkohole
vorzugsweise
a) 1- 20 Gew. % Fruchtsäureester,
b) 10 - 50 Gew. % anionische und/oder nichtionische und/oder kationische und/oder amphotere und/oder zwitterionische Tenside und
c) 0,5 - 5 Gew. % Fettalkohole
bevorzugter
a) 0,5 - 25 Gew. % Fruchtsäureester,
b) 5 - 70 Gew. % anionische und/oder nichtionische und/oder kationische und/oder amphotere und/oder zwitterionische Tenside und
c) 0,5 - 5 Gew. % Fettalkohole
mit der Maßgabe, dass sich die Mengenangaben mit Wasser und gegebenenfalls üblichen Hilfs- und Zusatzstoffen zu 100 Gew.-% ergänzen.

Die kosmetischen und/oder pharmazeutischen Zubereitungen, wie beispielsweise Haarshampoos, Duschbäder, Schaumbäder, Waschlotionen und dergleichen, können als weitere Hilfs- und Zusatzstoffe Emulgatoren, Perlglanzwachse, Konsistenzgeber, Verdickungsmittel, Polymere, Siliconverbindungen, Lecithine, Phospholipide, biogene Wirkstoffe, Antioxidantien, Antischuppenmittel, Filmbildner, Hydrotrope, Solubilisatoren, Konservierungsmittel, Parfümöle, Farbstoffe und dergleichen enthalten.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol(Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

➢ Ethylenoxidanlagerungsprodukte
   Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
➢ Partialglyceride
   Typische Beispiele für geeignete Partialglyceride sind Hydroxystearinsäuremonoglycerid, Hydroxystearinsäurediglycerid, lsostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
➢ Sorbitanester
   Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
➢ Anionische Emulgatoren
   Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
➢ Amphothere und kationische Emulgatoren
   Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl* Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Polymere

Geeignete kationische Polymere sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte VinylpyrrolidonNinylimidazol-Polymere, wie z.B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise VinylacetatlCrotonsäure-Copolymere, vinylpyrrolidon/Ninylacrylat-Copolymere, Vinylacetat/Butylmaleat/ Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, vinylpyrrolidon/vinylacetat-Copolymere, Vinylpyrrolidon/ Dimethylaminoethylmethacrylat/vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, lso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.l.42051), Indigotin (C.l.73015), Chlorophyllin (C.l.75810), Chinolingelb (C.l.47005), Titandioxid (C.l.77891), Indanthrenblau RS (C.l. 69800) und Krapplack (C.l.58000). Als Lumineszenzfarbstoff kann auch Luminol enthalten sein. Diese Farbstoffe werden üblicherweise in Konzentrationen von 0,001 bis 0,1 Gew.%, bezogen auf die gesamte Mischung, eingesetzt.

### Beispiele

### I. Rezepturbeispiele

Mengenangaben in Gew. %, Viskositätsmessung: Brookfield RVT, Spindel Nr. 4 bei 10 Upm, bei RT 23 ± 2°C

**Tabelle 1a**

| **Zubereitungen zur flüssigen Verarbeitung (Prozentangaben als Gew.-%) - Aussehen trüb** | | | | |
|---|---|---|---|---|
| **Handelsname** | **INCI-Name** | **1** | **2** | **3** |
| Cutina® KE 3602 | Citronensäure-trioctadecylester | 25,0 | 25,0 | 20,0 |
| Texapon® NSO | Sodium Laureth Sulfate | - | 20,0 | - |
| Plantacare® 1200 UP | Laurylglycoside | 20,0 | - | - |
| Dehydol® LS 4 DEO | Fatty alcohol C₁₂₋₁₄ + 4 EO | 5,0 | 5,0 | - |
| Dehyton® PK 45 | Cocamidopropyl Betaine | 20,0 | - | - |
| Dehyton® MC | Sodium Cocoamphoacetate | - | - | 77,45 |
| Lorol ®C18 | Fatty alcohol C₁₈ | 0,20 | 0,20 | 0,20 |
| Citronensäure 50 %ig | | 1,5 | 0,2 | 2,0 |
| Benzoesäure | | 0,35 | 0,35 | 0,35 |
| Wasser | | ad 100,0 | ad 100,0 | ad 100,0 |
| | | | | |
| pH-Wert | | 4,2 | 3,3 | 4,5 |
| Viskosität [mPa*s] | | 7700 | 9600 | 12000 |

**Tabelle 1b**

| **Zubereitungen zur flüssigen Verarbeitung (Prozentangaben als Gew.-%) - Aussehen trüb** | | | | |
|---|---|---|---|---|
| **Handelsname** | **INCI-Name** | **4** | **5** | **6** |
| Cutina® KE 3580 | Äpfelsäure-dioctadecylester | 25,0 | 25,0 | 20,0 |
| Texapon® NSO | Sodium Laureth Sulfate | - | 20,0 | - |
| Plantacare® 1200 UP | Laurylglycoside | 20,0 | - | - |
| Dehydol® LS 4 DEO | Fatty alcohol C₁₂₋₁₄ + 4 EO | 5,0 | 5,0 | - |
| Dehyton® PK 45 | Cocamidopropyl Betaine | 20,0 | - | - |
| Dehyton® MC | Sodium Cocoamphoacetate | - | - | 77,45 |
| Lorol ®C18 | Fatty alcohol C₁₈ | 0,20 | 0,20 | 0,20 |
| Citronensäure 50 %ig | | 1,5 | 0,2 | 2,0 |
| Benzoesäure | | 0,35 | 0,35 | 0,35 |
| Wasser | | ad 100,0 | ad 100,0 | ad 100,0 |
| | | | | |
| pH-Wert | | 4,2 | 3,3 | 4,5 |
| Viskosität [mpa*s] | | 7100 | 9500 | 11800 |

**Tabelle 2a**

| **Zubereitungen zur flüssigen Verarbeitung (Prozentangaben als Gew.-%) - gelartig, Aussehen transparent** | | | | |
|---|---|---|---|---|
| **Handelsname** | **INCI-Name** | **7** | **8** | **9** |
| Cutina® KE 3602 | Citronensäure-trioctadecylester | 20,0 | 23,0 | 20,0 |
| Eutanol G | 2- Octyl-dodecanol | - | - | 4,0 |
| Glycerin | | 5,0 | 15,0 | 10,0 |
| Texapon® NSO | Sodium Laureth Sulfate | - | 5,0 | - |
| Plantacare® 1200 UP | Laurylglycoside | 28,0 | - | 15,0 |
| Dehydol® LS 4 DEO | Fatty alcohol C₁₂₋₁₄ + 4 EO | - | 15,0 | - |
| Dehyton® PK 45 | Cocamidopropyl Betaine | - | - | 15,0 |
| Dehyton® MC | Sodium Cocoamphoacetate | - | - | 5,0 |
| Lorol ®C18 | Fatty alcohol C₁₈ | 0,20 | 0,20 | 0,20 |
| Citronensäure 50 %ig | | 3,5 | 0,5 | 0,5 |
| Benzoesäure | | 0,35 | 0,35 | 0,35 |
| Wasser | | ad 100,0 | ad 100,0 | ad 100,0 |
| | | | | |
| pH-Wert | | 4,2 | 3,3 | 4,5 |
| Viskosität [mPa*s] | | 25500 | 33100 | 38800 |

**Tabelle 2b**

| **Zubereitungen zur flüssigen Verarbeitung (Prozentangaben als Gew.-%) - gelartig, Aussehen transparent** | | | | |
|---|---|---|---|---|
| **Handelsname** | **INCI-Name** | **10** | **11** | **12** |
| Cutina® KE 3580 | Äpfelsäure-dioctadecylester | 20,0 | 23,0 | 20,0 |
| Eutanol G | 2- Octyl-dodecanol | - | - | 4,0 |
| Glycerin | | 5,0 | 15,0 | 10,0 |
| Texapon® NSO | Sodium Laureth Sulfate | - | 5,0 | - |
| Plantacare® 1200 UP | Laurylglycoside | 28,0 | - | 15,0 |
| Dehydol® LS 4 DEO | Fatty alcohol C₁₂₋₁₄ + 4 EO | - | 5,0 | - |
| Dehyton® PK 45 | Cocamidopropyl Betaine | - | - | 15,0 |
| Dehyton® MC | Sodium Cocoamphoacetate | - | - | 5,0 |
| Lorol ®C18 | Fatty alcohol C₁₈ | 0,20 | 0,20 | 0,20 |
| Citronensäure 50 %ig | | 1,5 | 0,2 | 2,0 |
| Benzoesäure | | 0,35 | 0,35 | 0,35 |
| Wasser | | ad 100,0 | ad 100,0 | ad 100,0 |
| | | | | |
| pH-Wert | | 4,2 | 3,3 | 4,5 |
| Viskosität [mPa*s] | | 25200 | 31800 | 39600 |

**Tabelle 3**

| | | | | |
|---|---|---|---|---|
| **Einsatz der Fruchtwachsdispersion in Shampoo und Duschbad-Formulierungen (Mengenangaben in Gew. %)** | | | | |

| **Handelsname** | **INCI-Name** | **V1** | **1** | **2** |
|---|---|---|---|---|
| Texapon®NSO | Sodium Laureth Sulfate | 35,0 | 35,0 | 35,0 |
| Dehyton® MC | Sodium Cocoamphoacetate | 8,0 | - | - |
| Wachshaltiges Compound Rezeptur 3, Tabelle 1a | | - | 10,0 | - |
| Wachshaltiges Compound Rezeptur 6, Tabelle 1 b | | - | - | 10,0 |
| Cosmedia Guar® C 261 N | Guar Gum | 0,2 | 0,2 | 0,2 |
| Parfümöl | | 0,3 | 0,3 | 0,3 |
| Euxyl® K | Konservierungsmittel | 0,1 | 0,1 | 0,1 |
| Natriumchlorid | | 0,85 | 1,2 | 0,95 |
| Wasser | | ad 100,0 | ad 100,0 | ad 100,0 |
| Citronensäure | zur pH-Einstellung | | | |
| | | | | |
| *pH-Wert* | | 5,5 | 5,4 | 5,4 |
| *Viskosität [mPa*s]* | | 5100 | 5500 | 6000 |

| ***Sensory Assessment*** | | | | |
|---|---|---|---|---|
| Haar | *Kämmbarkeit* | ◇ | ++ | +++ |
| | *Belastung* | ◇ | +++ | ++ |
| | *Elektrostatische Aufladung* | - | +++ | ++ |
| | *Frisierbarkeit* | -- | ++ | +++ |
| Haut | *Schaum Cremigkeit* | ◇ | +++ | ++ |
| | *Hautgefühl* | + | ++ | ++ |
| | *Feuchtigkeit* | - | +++ | ++ |
| | *TEWL [mglcm²*/*n]* | 4,1 | 1,2 | 1,4 |

| | | | | |
|---|---|---|---|---|
| Eigenschaften: (- -) sehr schlecht, (-) schlecht, (0) neutral, (+) gut, (++) sehr gut, (+++) ausnehmend gut TEWL-Werte : (> 6) schlecht, (6 - 4) normal, (2 - 4) gut, (< 2) sehr gut | | | | |

## Patentansprüche

1. Kosmetische und/oder pharmazeutische Zubereitungen, enthaltend
(a) 0,1 bis 30 Gew.-% Fruchtsäureester, ausgewählt aus Trialkylester der Citronensäure und Dialkylester der Äpfelsäure,
(b) 0,1 bis 90 Gew.-% anionische und/oder nichtionische und/oder kationische und/oder amphotere und/oder zwitterionische Tenside und
(c) 0,1 bis 10 Gew.-% Fettalkohole
mit der Maßgabe, dass sich die Mengenangaben mit Wasser und gegebenenfalls üblichen Hilfs- und Zusatzstoffen zu 100 Gew.-% ergänzen.

2. Zubereitungen nach Anspruch 1, **dadurch gekennzeichnet, dass** sie Fruchtsäurealkylester enthalten, deren Alkoholkomponente aus einen Alkanolrest mit 8 bis 22 Kohlenstoffatomen besteht.

3. Zubereitungen nach den Ansprüchen 1 bis 2, **dadurch gekennzeichnet, dass** sie Fruchtsäurealkylester enthalten, deren Alkoholkomponente aus einen Alkanolrest mit 16 bis 18 Kohlenstoffatomen besteht.

4. Zubereitungen nach den Ansprüchen 1 bis 3, **dadurch gekennzeichnet, dass** sie Tenside enthalten ausgewählt aus der Gruppe, die gebildet wird von Seifen, Alkylbenzolsulfonaten, Alkansulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Glycerinethersulfaten, Fettsäureethersulfaten, Hydroxymischethersulfaten, Monoglycerid(ether)sulfaten, Fettsäureamid(ether)-sulfaten, Mono- und Dialkylsulfosuccinaten, Mono- und Dialkylsulfosuccinamaten, Sulfotriglyceriden, Amidseifen, Ethercarbonsäuren und deren Salzen, Fettsäureisethionaten, Fettsäuresarcosinaten, Fettsäuretauriden, N-Acylaminosäuren, wie beispielsweise Acyllactylaten, Acyltartraten, Acylglutamaten und Acyl-aspartaten, Alkyloligoglucosidsulfaten, Proteinfettsäurekondensaten (insbesondere pflanzliche Produkte auf Weizenbasis) und Alkyl(ether)phosphaten, Fettalkoholpolyglycolethern, Alkylphenolpolyglycolethern, Fettsäurepolyglycolestern, Fettsäureamidpolyglycolethem, Fettaminpolyglycolethem, alkoxylierten Triglyceriden, Mischethern, Mischformalen, gegebenenfalls partiell oxidierte Alk(en)yloligoglykosiden bzw. Glucoronsäurederivaten, Fettsäure-N-alkylglucamiden, Proteinhydrolysaten (insbesondere pflanzliche Produkte auf Weizenbasis), Polyolfettsäureestern, Zuckerestern, Sorbitanestern, Polysorbaten, Aminoxiden, quartäre Ammoniumverbindungen, Esterquats, Alkylbetaine, Alkylamidobetaine, Aminopropionate, Aminoglycinate, Imidazoliniumbetaine und Sulfobetainen.

5. Zubereitungen nach den Ansprüchen 1 bis 4, **dadurch gekennzeichnet, dass** sie als nichtionische Tenside Alkyl- und/oder Alkenyloligoglykoside enthalten.

6. Zubereitungen nach den Ansprüchen 1 bis 5, **dadurch gekennzeichnet, dass** sie Fettalkohole enthalten, die 10 bis 18 Kohlenstoffatome aufweisen.

7. Zubereitungen nach den Ansprüchen 1 bis 6, **dadurch gekennzeichnet, dass** sie Fettalkohole enthalten, die 12 bis 14 Kohlenstoffatome aufweisen.

## Claims

1. Cosmetic and/or pharmaceutical preparations containing
(a) 0.1 to 30% by weight fruit acid esters selected from trialkyl esters of citric acid and dialkyl esters of malic acid,
(b) 0.1 to 90% by weight anionic and/or nonionic and/or cationic and/or amphoteric and/or zwitterionic surfactants and
(c) 0.1 to 10% by weight fatty alcohols,
with the proviso that the quantities shown add up to 100% by weight with water and optionally typical auxiliaries and additives.

2. Preparations as claimed in claim 1, **characterized in that** they contain fruit acid esters of which the alcohol component is an alkanol radical having 8 to 22 carbon atoms.

3. Preparations as claimed in claims 1 and 2, **characterized in that** they contain fruit acid alkyl esters of which the alcohol component is an alkanol radical having 16 to 18 carbon atoms.

4. Preparations as claimed in claims 1 to 3, **characterized in that** they contain surfactants selected from the group consisting of soaps, alkyl benzenesulfonates, alkanesulfonates, olefin sulfonates, alkylether sulfonates, glycerol ether sulfonates, α-methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic-acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligo-glucoside sulfates, protein fatty acid condensates (particularly wheat-based vegetable products) and alkyl (ether) phosphates, fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates, amine oxides, quaternary ammonium compounds, esterquats, alkylbetaines, alkylamidobetaines, aminopropionates, aminoglycinates, imidazolinium betaines and sulfobetaines.

5. Preparations as claimed in claims 1 to 4, **characterized in that** they contain alkyl and/or alkenyl oligoglycosides as nonionic surfactants.

6. Preparations as claimed in claims 1 to 5, **characterized in that** they contain fatty alcohols having 10 to 18 carbon atoms.

7. Preparations as claimed in claims 1 to 6, **characterized in that** they contain fatty alcohols having 12 to 14 carbon atoms.

## Revendications

1. Préparations cosmétiques et/ou pharmaceutiques, contenant
(a) 0,1 à 30 % en poids d'esters d'acides de fruits, choisis parmi les esters trialkyliques de l'acide citrique et les esters dialkyliques de l'acide malique,
(b) 0,1 à 90 % en poids de tensioactifs anioniques et/ou non ioniques et/ou cationiques et/ou amphotères et/ou zwitterioniques, et
(c) 0,1 à 10 % en poids d'alcools gras,
à condition que le total des quantités indiquées soit égal à 100 % en poids compte tenu de l'ajout d'eau et, éventuellement, d'additifs et d'adjuvants conventionnels.

2. Préparations selon la revendication 1,
**caractérisées en ce qu'**
elles contiennent des esters alkyliques d'acides de fruits dont les composants alcool se composent d'un radical alcanol comptant 8 à 22 atomes de carbone.

3. Préparations selon les revendications 1 et 2,
**caractérisées en ce qu'**
elles contiennent des esters alkyliques d'acides de fruits dont les composants alcool se composent d'un radical alcanol comptant 16 à 18 atomes de carbone.

4. Préparations selon les revendications 1 à 3,
**caractérisées en ce qu'**
elles contiennent des tensioactifs choisis dans le groupe constitué par les savons, sulfonates d'alkylbenzène, sulfonates d'alcanes, sulfonates d'oléfines, sulfonates d'alkyléther, sulfonates d'éther glycérique, sulfonates d'α-méthyl-ester, acides gras sulfonés, sulfates d'alkyle, éthersulfates d'alcools gras, éthersulfates de glycérol, éthersulfates d'acides gras, hydroxyéthersulfates mixtes, (éther)sulfates de monoglycérides, (éther)sulfates d'amides d'acides gras, sulfosuccinates de mono- et dialkyle, sulfosuccinamates de mono- et dialkyle, sulfotriglycérides, savons d'amides, acides éthercarboxyliques et leurs sels, iséthionates d'acides gras, sarcosinates d'acides gras, taurides d'acides gras, N-acylaminoacides, comme par exemple les acyllactylates, les acyltartrates, les acylglutamates et les acylaspartates, sulfates d'alkyloligoglucosides, condensats d'acides gras de protéines (notamment produits végétaux à base de blé) et phosphates (d'éther) d'alkyle, éthers polyglycoliques d'alcools gras, éthers polyglycoliques d'alkylphénol, esters polyglycoliques d'acides gras, éthers polyglycoliques d'amides d'acides gras, éthers polyglycoliques d'amines grasses, triglycérides alcoxylés, éthers mixtes, formals mixtes, dérivés de l'acide glucoronique ou oligoglycosides d'alkyle ou d'alcényle éventuellement partiellement oxydés, N-alkylglucamides d'acides gras, hydrolysats de protéines (notamment produits végétaux à base de blé), esters d'acides gras et de polyols, esters de sucre, esters de sorbitan, polysorbates, oxydes aminés, composés d'ammonium quaternaires, esterquats, alkylbétaïnes, alkylamidobétaïnes, aminopropionates, aminoglycinates, imidazolinium bétaïnes et sulfobétaïnes.

5. Préparations selon les revendications 1 à 4,
**caractérisées en ce qu'**
elles contiennent comme tensioactifs non ioniques des oligoglycosides d'alkyle et/ ou d'alcényle.

6. Préparations selon les revendications 1 à 5,
**caractérisées en ce qu'**
elles contiennent des alcools gras présentant 10 à 18 atomes de carbone.

7. Préparations selon les revendications 1 à 6,
**caractérisées en ce qu'**
elles contiennent des alcools gras présentant 12 à 14 atomes de carbone.
